Europäisches Patentamt

European Patent Office

Office européen des brevets

(11) Veröffentlichungsnummer: **0 418 682 A1**

## (12) EUROPÄISCHE PATENTANMELDUNG

(21) Anmeldenummer: 90117347.6

(22) Anmeldetag: 08.09.90

(51) Int. Cl.5: **B01J 35/06**, D01F 11/12, C07C 209/36

(30) Priorität: 21.09.89 DE 3931420

(43) Veröffentlichungstag der Anmeldung: 27.03.91 Patentblatt 91/13

(84) Benannte Vertragsstaaten: BE CH DE FR GB IT LI NL

(71) Anmelder: BAYER AG

W-5090 Leverkusen 1 Bayerwerk(DE)

(72) Erfinder: Sirinyan, Kirkor, Dr.
Humperdinckstrasse 12
W-5060 Bergisch Gladbach 2(DE)
Erfinder: Immel, Otto, Dr.
Immenhofweg 21
W-4150 Krefeld(DE)
Erfinder: Schwarz, Hans-Helmut, Dr.
Rather Strasse 90
W-4150 Krefeld(DE)
Erfinder: Eickmans, Johannes, Dr.
Sandstrasse 33
W-4000 Duesseldorf 12(DE)
Erfinder: Waldmann, Helmut, Dr.
Henry-T.-von Boettinger-Strasse 15
W-5090 Leverkusen 1(DE)

(54) Katalysatoren auf der Basis von metalldotierten Kohlenstoffasern, deren Herstellung und deren Verwendung.

(57) Neue Katalysatoren auf der Basis von metalldotierten Kohlefasern sind dadurch gekennzeichnet, daß sie Kohlefasern mit einer spezifischen Oberfläche von weniger als 25 $m^2/g$ enthalten. Solche Katalysatoren können mit Vorteilen zur Herstellung von gegebenenfalls Alkyl-substituierten Anilinen durch Hydrierung von gegebenenfalls Alkyl-substituierten Nitrobenzolen verwendet werden.

EP 0 418 682 A1

# KATALYSATOREN AUF DER BASIS VON METALLDOTIERTEN KOHLENSTOFFASERN, DEREN HERSTELLUNG UND DEREN VERWENDUNG

Die vorliegende Erfindung betrifft Katalysatoren auf der Basis von metalldotierten Kohlenstoffasern mit niedriger Oberfläche, deren Herstellung und ein Verfahren zur Herstellung von Anilinen unter Verwendung solcher Katalysatoren.

Übliche Trägerkatalysatoren enthalten Metalle oder Metallverbindungen z.B. auf Aluminiumoxiden, Titanoxiden, Magnesiumoxiden, Spinellen oder Zeolithen (siehe z.B. J. Catal. 112 , Seiten 145, 183 und 282 (1988) und J. Phys. Chem. 89 , 4267 (1985)). Ihre Herstellung erfolgt normalerweise, indem man Lösungen einfacher Metallsalze auf das Trägermaterial auftränkt, das Lösungsmittel entfernt und das so niedergeschlagene Metallsalz gegebenenfalls zum Metall reduziert. Nachteilig bei solchen Katalysatoren sind ihre geringe Wärmeleitfähigkeit von ca. 0,1 bis 1 W/m.K (was besondere Maßnahmen zur Ableitung der Reaktionswärme erfordert), ihre große Schüttdichte (was in Reaktoren zu großen Druckverlusten führt), ihre geringe mechanische Festigkeit und ihre geringe Abriebfestigkeit.

Es sind auch schon Trägerkatalysatoren bekannt geworden, die Metalle oder Metallverbindungen auf Kohlefasern enthalten (siehe z.B. JP-OS'en 53-014 665, 53-014 666 und 57-144 034, DE-OS'en 2 345 297, 2 750 282 und 3 217 299 und US-PS 3 297 490). Bei allen diesen Katalysatoren kamen Kohlefasern zum Einsatz, die durch eine Wärmebehandlung aktiviert worden waren und deshalb spezifische Oberflächen von über 500 m²/g aufwiesen. Solche Kohlefasern sind mit den Lösungen von einfachen Metallsalzen gut zu imprägnieren. Es werden jedoch größere Metallmengen benötigt, um eine technisch ausreichende katalytische Aktivität zu erhalten. Außerdem sind solche Katalysatoren wenig abriebfest und wenig mechanisch belastbar.

Es wurden nun Katalysatoren auf der Basis von metalldotierten Kohlefasern gefunden, die dadurch gekennzeichnet sind, daß sie Kohlefasern mit einer spezifischen Oberfläche von weniger als 25 m²/g enthalten.

Für die vorliegende Erfindung geeignete Kohlefasern können beispielsweise aus Polyamid-, Polyvinylchlorid-, Celluloseacetat- oder Polyacrylnitril-Fasern hergestellt werden, wobei auch Mischungen und/oder Copolymerisate dieser Materialien in Frage kommen. Bevorzugt sind Kohlefasern, die aus Polyacrylnitrilfasern hergestellt worden sind. Der Durchmesser der Kohlefasern kann beispielsweise im Bereich 0,5 bis 150 μm liegen, bevorzugt liegt er im Bereich 5 bis 15 μm. Die Länge der Kohlefasern kann beispielsweise zwischen 2 μm und ∞ ( = Endlosfaser) liegen. Erfindungsgemäß beträgt die spezifische Oberfläche der Kohlefasern weniger als 25 m²/g. Beispielsweise kann sie zwischen 0,1 und 20 m²/g liegen, vorzugsweise zwischen 0,2 und 18 m²/g. Die Herstellung solcher Kohlefasern ist an sich bekannt (siehe z.B. International Fiber Science and Technology Series, Vol. 3, "Carbon Fibers", J.B. Donnet and R.CH. Barsal, Marcel Dekker-Verlag New York 1984)). Derartige Kohlefasern werden bisher technisch als Verstärkungsfasern für Kunststoffe hergestellt und verwendet.

Als Metalle zur Dotierung der Kohlefasern kommen z.B. diejenigen der 1. und 8. Nebengruppe des Periodensystems der Elemente in Frage. Bevorzugt sind Kupfer, Silber, Gold, Ruthenium, Rhodium, Palladium und/oder Platin.

Die Dotierung erfolgt so, daß man zunächst eine Komplexverbindung des für die Dotierung vorgesehenen Metalls oder Komplexverbindungen der für die Dotierung vorgesehenen Metalle herstellt.

Als Liganden für solche Komplexe kommen vorzugsweise solche in Frage, die über die zur Metallbindung erforderlichen Gruppen hinaus wenigstens eine weitere funktionelle Gruppe enthalten. Beispiele für solche funktionellen Gruppen sind: Carbonsäure-, Carbonsäurehalogenid-, Carbonsäureanhydrid-, Carbonsäureester-, Carbonsäureamid-, Carbonsäureimid-, Aldehyd-, Keton-, Ether-, Sulfonamid-, Sulfonsäure-, Sulfonat-, Sulfonsäurehalogenid-, Sulfonsäureester-, Vinylsulfonsäure-, Acrylsäure-, Amino-, Hydroxyl-, Isocyanat-, Olefin-, Acetylen-, Mercapto- und Epoxidgruppen sowie halogen-haltige Heterocyclen, längere Alkyl- und Alkylenreste (ab 8 Kohlenstoffatomen) und Phenylgruppen. Solche weiteren funktionellen Gruppen erhöhen im allgemeinen die Haftfestigkeit der Metalle bzw. Metallkomplexe auf der Faseroberfläche.

Gegebenenfalls kann man durch einfache Vorversuche feststellen, welche funktionellen Gruppen auf welchen Kohlefasern besonders gute Haftfestigkeiten ergeben. Wenn man aus Polyacrylnitrilfasern hergestellte Kohlefasern einsetzt, so ergeben Komplexe mit längeren Alkyl-, Alkenyl-und/oder Phenylgruppen im allgemeinen gleichmäßige und abriebfeste Beschichtungen. Für eine gleichmäßige Verankerung der Komplexe auf der Faseroberfläche sind häufig Komplexe mit Carbonsäure-, Carbonsäureanhydrid-, Ether-, Alkyl-, Alkenyl- und/oder Phenylgruppen vorteilhaft.

Bei den für die Metallbindung erforderlichen Gruppen der Komplexliganden kann es sich z.B. um Kohlenstoff-Kohlenstoff und Kohlenstoff-Stickstoff Doppel- oder Dreifachbindungen oder um Gruppen handeln, die einen Chelatkomplex ausbilden können, z.B. OH-, SH-, CO-, CS- oder COOH-Gruppen oder, insbesondere um α,β-ungesättigte Ketone.

Die Metallkomplexe werden dann in einem Lösungsmittel gelöst oder darin dispergiert oder damit angerieben und in dieser Form auf die Kohlefasern aufgebracht:

Wenn der Metallkomplex Liganden enthält, die eine chemische Fixierung auf der Substratoberfläche ermöglichen, kann er auch aus wäßriger Phase aufgebracht werden

Es ist vorteilhaft, beim Zusammenbringen von Lösungsmittel und Metallkomplex auf folgendes zu achten:

- Die verwendeten Metallkomplexe sollten an der Luft und gegenüber Feuchtigkeit stabil sein. Sie sollten in organischen Lösungsmitteln gut löslich und in Wasser wenig löslich sein. Vorzugsweise sollten sie mit gebräuchlichen Reduktionsmitteln zum Metall reduzierbar sein.
- Lösungen von Metallkomplexen in organischen Lösungsmitteln sollten an der Luft und gegenüber Feuchtigkeit stabil sein.
- Das organische Lösungsmittel sollte leicht entfernbar sein.
- Bei der gegebenenfalls durchzuführenden Reduktion der Metallkomplexe sollten keine Stoffe frei werden, die Katalysatorsysteme vergiften.

Sofern das Metall in reduzierter Form vorliegt, sollte es so fest an der Faseroberfläche haften, daß es durch in Kontakt bringen mit wäßrigen Lösungen nicht entfernt wird

Anschließend an das Aufbringen der Komplexverbindung auf die Kohlenstoffasern kann sie gegebenenfalls zum Metall reduziert werden. Das Aufbringen der Komplexverbindung wird vorzugsweise folgendermaßen durchgeführt: Eine Komplexverbindung eines Elements der 1. oder 8. Nebengruppe des Periodensystems der Elemente, insbesondere von Kupfer, Silber, Gold, Ruthenium, Rhodium, Palladium oder Platin, die mindestens eine zusätzliche funktionelle Gruppe enthält, wird in einem organischen Lösungsmittel gelöst. Es können auch Mischungen von verschiedenen Komplexverbindungen eingesetzt werden. Die Konzentration der Komplexverbindung kann z.B. zwischen 0,01 und 10 g/l betragen, sie kann in besonderen Fällen auch darunter oder darüber liegen.

Als organische Lösungsmittel sind polare, protische und aprotische Lösungsmittel bevorzugt, wie Methylenchlorid, Chloroform, 1,1,1-Trichlorethan, Trichlorethylen, Perchlorethylen, Aceton, Methylethylketon, Butanol, Ethylenglykol und Tetrahydrofuran. Es können auch Gemische dieser Lösungsmittel untereinander und/oder Gemische dieser Lösungsmittel mit anderen Lösungsmitteln, beispielsweise mit Benzin, Ligroin oder Toluol, verwendet werden.

Mit diesen Lösungen werden die Oberflächen der Kohlenstoffasern benetzt. Die Einwirkungsdauer beträgt vorzugsweise 1 sec bis 10 min. Besonders geeignet sind dazu Verfahren wie das Eintauchen des Substrats in die Lösungen oder Besprühen der Substratoberflächen mit den Komplexlösungen. Es ist auch möglich, die Komplexlösungen durch Stempel oder durch Druckverfahren aufzubringen.

Das Aufbringen der Metallkomplexe kann z.B. bei Temperaturen von -20 bis +100°C durchgeführt werden. Relativ niedrige Temperaturen sind bei niedrigsiedenden Lösungsmitteln und chemisch leicht angreifbaren Substraten bevorzugt, relativ höhere Temperaturen bei chemisch resistenten Substraten. In Ausnahmefällan kann auch bei niedrigereren oder höheren Temperaturen als -20 oder +100°C gearbeitet werden. Besonders bevorzugt sind Temperaturen von 0 bis 80°C.

Nach der Benetzung wird das organische Lösungsmittel entfernt. Niedrigsiedende Lösungsmittel bevorzugt durch Verdampfen, z.B im Vakuum. Bei höhersiedenden Lösungsmitteln sind andere Verfahren bevorzugt, z.B. die Extraktion mit einem Lösungsmittel, in dem der jeweilige Metallkomplex unlöslich ist.

Die so beschichteten Kohlenstoffasern können anschließend gegebenenfalls mit einem Reduktionsmittel, z.B. mit Hydrazinhydrat, Wasserstoff, Formaldehyd, Hypophosphit oder Dimethylaminoboran behandelt werden, wobei dann der Metallkomplex sich in das jeweilige Metall umwandelt

Die auf die Kohlefasern aufgebrachte Metallmenge kann beispielsweise im Bereich von 0,001 bis 2,5 Gew.-% variieren. Vorzugsweise liegt diese Menge im Bereich von 0,1 bis 1,0 Gew.-%.

Die erfindungsgemäßen Katalysatoren können zur Durchführung von verschiedenen katalysierten Reaktionen eingesetzt werden Beispiele sind die Hydrierung von aliphatischen und aromatischen Nitroverbindungen, von C-C- Mehrfachbindungen und von Carbonylgruppen. Vorzugsweise werden sie zur Hydrierung von Nitrobenzol zu Anilin und von alkylierten Nitrobenzolen zu den entsprechenden Anilinen verwendet.

Die neuen Katalysatoren zeichnen sich durch eine hervorragende Wärmeleitfähigkeit im Bereich 2 bis 5 W/m.K, durch gute Chemikalienbeständigkeit und gute Abriebfestigkeit aus. Durch ihren Einsatz werden die nicht erwünschten örtlichen Überhitzungen (sog. "hot spots") vermieden.

Die neuen Katalysatoren sind z.B. in Form von Geweben, Vliesen, Stapelfasern oder Endlosfasern einsetzbar. Dadurch verringert sich zusätzlich der Druckverlust in mit solchen Katalysatoren gefüllten Reaktoren.

Beispiele

Beispiel 1

50 g Kohlefasern, hergestellt aus Polyacrylnitril, bezogen von der Firma Sigri GmbH, 8901 Meitingen, Bundesrepublik Deutschland, mit Längen von ca. 60 mm, einem Durchmesser von 8 ±1 $\mu$m und einer spezifischen Oberfläche von 0,14 m²/g wurden bei Raumtemperatur in n-Hexan vorgereinigt, getrocknet und dann in ein Bad getaucht, das aus 1000 ml frisch destilliertem Tetrahydrofuran und 2,678 g Bisbenzonitrilpalladium-II-dichlorid bestand. Anschließend wurden die Fasern an der Luft getrocknet und in einer Reduktionslösung, bestehend aus 20 ml Hydrazinhydrat, gelöst in 980 ml Wasser, bei Raumtemperatur im Verlaufe von 20 Minuten nachbehandelt. Es wurden palladiumdotierte Kohlefasern erhalten, an denen das Palladium gut haftete. Die Palladiummenge betrug 0,9 g Palladium pro kg Faser

Die so dotierten Kohlefasern wurden als Katalysator zur Herstellung von Anilin durch Hydrierung von Nitrobenzol eingesetzt. Dabei wurde in der Gasphase und unter Atmosphärendruck wie folgt gearbeitet: Ein 16 mm weites Glasrohr wurde mit 4,8 g wie vorstehend beschrieben hergestellten palladiumdotierten Kohlefasern von unten her beschickt. Der obere Teil des Reaktionsrohres wurde mit inerten Füllkörpern gefüllt. Das Glasrohr wurde mit einem Ölthermostat auf 28° C erhitzt und bei dieser Temperatur gehalten. Von oben wurde kontinuierlich Nitrobenzol mit einer kalibrierten Dosiervorrichtung eingespeist. Das Nitrobenzol verdampfte in dem Teil des Rohres, der mit Füllkörpern gefüllt war. Das Nitrobenzol strömte zusammen mit dem ebenfalls von oben eingeleiteten Wasserstoff (20 l/h) durch die Katalysatorschicht. Die Reaktionsdauer betrug insgesamt 1000 Stunden, wobei mehrfach der Durchsatz verändert wurde, d.h. verschiedene Katalysatorbelastungen eingestellt wurden. Die Ergebnisse sind in der nachfolgenden Tabelle 1 zusammengestellt. Daraus ist ersichtlich, daß bei einer Katalysatorbelastung von weniger als 1 g/g.h Nitrobenzol vollständig zu Anilin umgesetzt wurde (100 % Umsatz und 100 % Selektivität).

Tabelle 1

| Belastung [g/g.h] | gesamte Kat.-Laufzeit [h] | Umsatz [%] | Selektivität [%] |
|---|---|---|---|
| 0,879 | 68,5 | 100 | ~100 |
| 0,859 | 159,0 | 100 | ~100 |
| 0,914 | 223,5 | 100 | ~100 |
| 1,953 | 289,0 | 68,1 | 97,4 |
| 1,857 | 352,5 | 64,0 | 98,3 |
| 0,663 | 492,5 | 96,8 | ~100 |
| 0,918 | 604,5 | 98,7 | ~100 |
| 0,983 | 722,0 | 99,7 | ~100 |
| 0,479 | 881,5 | 100 | ~100 |
| 2,095 | 924,0 | 56,6 | 98,4 |
| 0,661 | 988,5 | 100 | ~100 |

Beispiel 2

50 g der auch in Beispiel 1 verwendeten Kohlefasern wurden in einem Bad bestehend aus 1000 ml Ethylenchlorid und 1,705 g Mesityloxidpalladium-II-chlorid, wie in Beispiel 1 beschrieben, mit Palladium dotiert und in einem Bad bestehend aus 30 g Formaldehyd und 970 ml Wasser bei Raumtemperatur 15 Minuten lang reduziert. Es wurden mit Palladium dotierte Kohlefasern erhalten mit einem Palladiumgehalt von 0,9 g/kg Fasermaterial. Das so dotierte Fasermaterial erwies sich als hervorragend abriebfest.

4

Die so dotierten Kohlefasern wurden in gleicher Weise wie in Beispiel 1 beschrieben zur Herstellung von Anilin durch Hydrierung von Nitrobenzol eingesetzt.

Die erzielten Ergebnisse sind aus der nachfolgenden Tabelle 2 ersichtlich.

Tabelle 2

| Belastung [g/g.h] | gesamte Kat.-Laufzeit [h] | Umsatz [%] | Selektivität [%] |
|---|---|---|---|
| 0,923 | 68,5 | 100 | ~100 |
| 0,948 | 159,0 | 100 | ~100 |
| 1,027 | 233,5 | 99,6 | ~100 |
| 2,068 | 289,0 | 71,1 | 98,3 |
| 2,109 | 352,5 | 71,6 | 98,9 |
| 1,269 | 492,5 | 83,5 | ~100 |
| 1,693 | 604,5 | 77,0 | 97,8 |
| 1,274 | 721,5 | 97,1 | ~100 |
| 1,359 | 881,0 | 97,3 | ~100 |
| 2,975 | 923,5 | 58,2 | 96,7 |
| 1,260 | 988,0 | 99,5 | ~100 |

Beispiel 3

50 g Endlos-Kohlefasern der Firma Sigri mit einer spezifischen geometrischen Oberfläche von 0,2 m$^2$/g wurden in einer Lösung bestehend aus 1000 ml Tetrachlorethylen und 1,8 g Hepten-3-on-2-palladium-II-chlorid in entsprechender Weise, wie in Beispiel 1 beschrieben, dotiert. Es wurden palladiumdotierte, abriebfeste Kohlefasern erhalten, die 0,85 g Palladium/kg Fasermaterial enthielten. Diese wurden mit praktisch gleichen Ergebnissen wie im Beispiel 1 in die dort beschriebene Herstellung von Anilin durch Hydrierung von Nitrobenzol eingesetzt.

Beispiel 4

50 g Kohlefasern wie auch im Beispiel 1 verwendet, wurden in einer Losung bestehend aus 2,0 g 1,2-Butadienpalladium-II-dichlorid und 1.000 ml Methylenchlorid in entsprechender Weise, wie in Beispiel 1 beschrieben, behandelt. Es wurden palladiumdotierte Kohlefasern (0,8 g Palladium/kg Fasermaterial) erhalten. Die Wärmeleitfähigkeit der Kohlefasern wurde durch die Dotierung nicht negativ beeinflußt. Die dotierten Kohlefasern wurden mit praktisch gleichen Ergebnissen wie in Beispiel 1 in die dort beschriebene Herstellung von Anilin durch Hydrierung von Nitrobenzol eingesetzt.

Beispiel 5

50 g der auch in Beispiel 1 verwendeten Kohlefasern wurden in einer Lösung bestehend aus 1,8 g Allylpalladium-II-chlorid und 1000 ml Methylenchlorid entsprechend der in Beispiel 1 beschriebenen Methodik behandelt und dann in einer Lösung bestehend aus 20 g Dimethylaminoboran, 1 g Natriumhydroxid und 370 ml Wasser nachbehandelt. Es wurden palladiumdotierte Kohlefasern erhalten, die mit praktisch den gleichen Ergebnissen wie im Beispiel 1 in die dort beschriebene Herstellung von Anilin durch Hydrierung von Nitrobenzol eingesetzt wurden.

Beispiel 6

In einem weiteren Hydrierversuch wurden 4,8 g Kohlefasern eingesetzt, die wie im Beispiel 1 beschrieben, mit Palladium beschichtet worden waren. Das Reaktionsrohr wurde auf 280 ° C gehalten. Der Nitrobenzoldurchsatz entsprach einer Katalysatorbelastung von 0,65 g/g•h. Das Reaktionsprodukt enthielt in den ersten 64,5 Stunden 97,1 % Anilin und 2,9 % nichtumgesetztes Nitrobenzol. Danach verlief die Reaktion mit vollständigem Umsatz und einer Anilinausbeute von über 99,9 %. Die Hydrierung wurde 1.857,5 Stunden betrieben. Während dieser Zeit wurde kein Aktivitätsverlust am Katalysator festgestellt.

**Ansprüche**

1. Katalysatoren auf der Basis von metalldotierten Kohlefasern, dadurch gekennzeichnet, daß sie Kohlefasern mit einer spezifischen Oberfläche von weniger als 25 m²/g enthalten.

2. Katalysator nach Anspruch 1, dadurch gekennzeichnet, daß die Kohlefasern aus Polyamid-, Polyvinylchlorid-, Celluloseacetat- oder Polyacrylnitril-Fasern hergestellt werden

3. Katalysatoren nach Ansprüchen 1 und 2, dadurch gekennzeichnet, daß die Kohlefasern aus Polyacrylnitril-Fasern hergestellt werden.

4. Katalysatoren nach Ansprüchen 1 bis 3, dadurch gekennzeichnet, daß die Kohlefasern Durchmesser im Bereich von 0,5 bis 150 μm und Längen zwischen 2 μm und unendlich aufweisen.

5. Verfahren zur Herstellung von Katalysatoren auf Basis von metalldotierten Kohlefasern, dadurch gekennzeichnet, daß man Kohlefasern mit einer spezifischen Oberfläche von weniger als 25 m²/g mit einem oder mehreren Metallen dotiert

6. Verfahren nach Anspruch 5, dadurch gekennzeichnet, daß man zur Dotierung ein Metall aus der 1. und/oder 8. Nebengruppe des Periodensystems der Elemente verwendet.

7. Verfahren nach Ansprüchen 1 bis 6, dadurch gekennzeichnet, daß man die Metalldotierung mit einer Komplexverbindung vornimmt, wobei der Komplex Liganden enthält, die über die zur Metallbindung erforderlichen Gruppen hinaus wenigstens eine weitere funktionelle Gruppe enthalten.

8. Verfahren nach Anspruch 7, dadurch gekennzeichnet, daß es sich bei den weiteren funktionellen Gruppen um Carbonsäure-, Carbonsäurehalogenid-, Carbonsäureanhydrid-, Carbonsäureester-, Carbonsäureamid-, Carbonsäureimid-, Aldehyd-, Keton-, Ether-, Sulfonamid-, Sulfonsäure-, Sulfonat-, Sulfonsäurehalogenid-, Sulfonsäureester-, Vinylsulfonsäure-, Acrylsäure-, Amino-, Hydroxyl-, Isocyanat-, Olefin-, Acetylen-, Mercapto- und/oder Oxidgruppen handelt, sowie um halogenhaltige Heterocyclen, Alkyl- und Alkenylreste mit mehr als 8 Kohlenstoffatomen oder Phenylgruppen.

9. Verfahren nach Anspruch 7, dadurch gekennzeichnet, daß sich an die Dotierung eine Reduktion anschließt.

10. Verwendung von Katalysatoren gemäß Anspruch 1 zur Hydrierung von gegebenenfalls alkylierten Nitrobenzolen zu den entsprechenden Anilinen.

Europäisches
Patentamt

**EUROPÄISCHER
RECHERCHENBERICHT**

Nummer der Anmeldung

**EP 90 11 7347**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | DE-A-2 345 297   (KANEBO) <br> – – – | | B 01 J 35/06 <br> D 01 F 11/12 <br> C 07 C 209/36 |
| A | FR-A-2 521 873   (ASAHI) <br> – – – | | |
| A | FR-A-2 280 430   (SHOEI) <br> – – – | | |
| A | US-A-4 122 040   (McCARROL et al.) <br> – – – – – | | |

| | RECHERCHIERTE SACHGEBIETE (Int. Cl.5) |
|---|---|
| | B 01 J <br> D 01 F |

Der vorliegende Recherchenbericht wurde für alle Patentansprüche erstellt

| Recherchenort | Abschlußdatum der Recherche | Prüfer |
|---|---|---|
| Den Haag | 14 Dezember 90 | LO CONTE C. |